# EUROPEAN PATENT APPLICATION

(11) **EP 3 906 852 A1**
(43) Date of publication of application: **10.11.2021**
(21) Application number: 21172545.2
(22) Date of filing: 06.05.2021
(51) Int. Cl.: A61B 5/117, A47K 11/02, A61B 5/00, A61B 5/0205, A61B 5/024, A61B 5/0245, A61B 5/0507, A61B 5/11, A61B 5/268, A61B 5/308, A61B 5/327

(54) **METHOD AND SYSTEM FOR BIOMETRIC RECOGNITION, IN PARTICULAR OF USERS OF SANITATION FACILITIES**

(30) Priority: 06.05.2020 IT 202000010102
(71) Applicant: OLI - SISTEMAS SANITARIOS, S.A., 3800-314 Aveiro (PT)
(72) Inventor: MOURA DE OLIVEIRA, Antonio Manuel, 3800-314 AVEIRO (PT); RODRIGUES DE ALMEIDA, Hugo Márcio, 3800-314 AVEIRO (PT); PLÁCIDO DA SILVA, Hugo Humberto, 3800-314 AVEIRO (PT)
(74) Representative: Cernuzzi, Daniele

(57) **Abstract**

A method for biometric recognition, in particular of a user of sanitation facilities, comprises the steps of:
- providing a contact measurement module (31) having at least one sensor (51) defining a direct interface with the user body for acquiring ECG signals from the user and comprising a pair of sensor terminals (52) so as to be in direct contact with the user body;
- detecting an electrical potential difference measured between said two sensor terminals (52) of the contact measurement module (31) ;
- processing in continuous mode the signals detected by the contact measurement module (31) to provide ECG data of the user;
- performing the biometric recognition of the user by comparing said ECG data with data stored in a database of known users, so as to identify a current user.

## Description

### CROSS-REFERENCE TO RELATED APPLICATIONS

This patent application claims priority from Italian patent application no. 102020000010102 filed on 06/05/2020.

### TECHNICAL FIELD

The present invention relates to a method and a system for biometric recognition, in particular of users of sanitation facilities.

### BACKGROUND ART

Biometric recognition of a user may be desirable in many situations. For example, a typical situation wherein a certain identification is required is in sanitation structures and facilities provided with automatic systems for detecting body parameters from the user.

Biomedical sensing has greatly evolved from traditional monolithical clinical setups, and many features can now be found in the form of wrist-worn wearable computers (aka wearables). Despite the rapid growth, wearables suffer from high abandon rates and are often described as a fad (or novelty item). Furthermore, users need to remember wearing the devices, charging them, and styling can be an issue too. As an alternative or as a complement, new approaches for biomedical sensing where no voluntary action from the user is needed are of utmost importance.

Therefore, so-called "off-the-person" (or "invisible") approach has been also proposed, based on the use of sensors which are integrated in everyday use objects. One important aspect in this approach is the mapping between the collected data and the monitored user, i.e. identification or authentication. The former are generally framed in the field of biometric recognition. Typical biometric traits are only suitable for momentary validation, bound to a short period of time, and require either recall (e.g. password), a token (e.g. identity card), or a physical (e.g. fingerprint) or behavioral (e.g. writing patterns) token from the user.

In a continuous biometric recognition and biomedical assessment paradigm, there are multiple constraints currently unmet within the state of the art. Especially, few practical solutions are known within the state-of-the-art that provide an effective end-to-end solution for this purpose. Nevertheless, several examples of systems that provide biomedical assessment in sanitation facilities, in particular associated with toilet seats, can already be found.

For example, US2016374619A1 discloses a system and a method for medical analysis of a subject seated on a toilet seat.

Other examples of similar systems and methods are provided by WO2005070288 and WO2018180398.

All the above mentioned prior art documents fail however to provide a fully effective and reliable system for detecting biomedical parameters from a subject, in particular without requiring an active intervention by the subject and at the same time ensuring the correct identification of the subject.

In fact, the prior art systems generally suffer from some drawbacks: for example, some devices are not able to provide a reliable identification of the users; and/or require several sensors and detection terminals that interface with the body of the user; and/or provide some parameters with a low level of accuracy and reliability; etc.

### DISCLOSURE OF INVENTION

It is an object of the present invention to provide a method and a system for biometric recognition, in particular of a user of sanitation facilities such as a subject seated on a toilet seat, which overcome the described drawbacks of the known art.

In particular, it is an object of the invention to provide a method and a system which provide reliable identification of the user together with fully effective and reliable data detection of biomedical parameter of the user.

The present invention therefore relates to a method and a system for biometric recognition as defined in basic terms in the appended claims 1 and 7, respectively.

Additional preferred features of the invention are indicated in the dependent claims.

The system and method of the invention are particularly advantageous since all the sensors make use of just one seamless interface with the user, which does not require metallic electrodes, and enables the intrinsic identification of the user through an integrated system that collects the data using methods that do not require placement of the sensor arrangement on the subject.

As previously mentioned, user identification is a particularly critical feature, in the sense that sanitary facilities are used by a multitude of individuals, and mapping both the extracted features and the results of the analysis to the correct individual is of utmost importance for the validity of clinical decisions.

Another particularly advantageous feature of the present invention is that the sensory unit uses a virtual ground in the embodiments that require direct contact with the user, enabling the use of only two contact points with the subject specifically for Electrocardiographic (ECG) data acquisition.

Other features of the invention, implemented in preferred embodiments, are also particularly advantageous with respect to the known systems.

In particular, the toilet seat is specifically designed to promote a high level of hygiene in the use of the system, as well as the identification of the user by using signals collected through non-metallic sensors integrated in the toilet seat.

Basically, the invention is based on use of a system comprising a control unit, a sensory unit, and a power supply unit; the system as a whole is designed to perform the measure of body parameters by detecting and processing signals from the user body, and to display related information representing biomedical parameters of: electrocardiography (ECG), photoplethysmography (PPG), functional near infrared spectroscopy (fNIRS), phonocardiography (PCG), inertial measurement unit (IMU), radio interferometry (RI), optical fiber (OF), video and/or thermal imaging (VTI).

The system of the invention is configured to perform biometric recognition and/or biomedical monitoring of users of sanitation facilities in a continuous way, even though it can perform the same function momentarily, in the same way performed by other biomedical sensing devices.

It is in any case clear that the method and system of the invention can be used in different situations and with different devices, not only in sanitation facilities. In fact, the invention can be applied in all those cases where users touch or are in proximity of (depending on the kind of sensors used) of any object in which the sensors can be housed.

### BRIEF DESCRIPTION OF THE DRAWINGS

Further characteristics and advantages of the present invention will become clear from the description of the following non-limitative embodiments, with reference to the figures in the accompanying drawings, in which:
- Figure 1 is a schematic view of a system for detecting biomedical parameters from a subject seated on a toilet seat, in accordance with a first embodiment of the invention, integrated in a toilet seat assembly for toilets or other sanitary facilities;
- Figure 2 is a block diagram showing the main components of the system of the invention;
- Figures 3 and 4 are a perspective view and a lateral view, respectively, of a sensor terminal for use in the system of the invention;
- Figures 5 and 6 are a perspective view and a lateral view, respectively, of a variation of the sensor terminal of Figures 3-4;
- Figure 7 is a perspective view of another variation of the sensor terminal of Figures 3-4;
- Figures 8 to 10 are respective schematic views of further embodiments of the system in accordance with the invention, integrated in a toilet seat assembly.

### BEST MODE FOR CARRYING OUT THE INVENTION

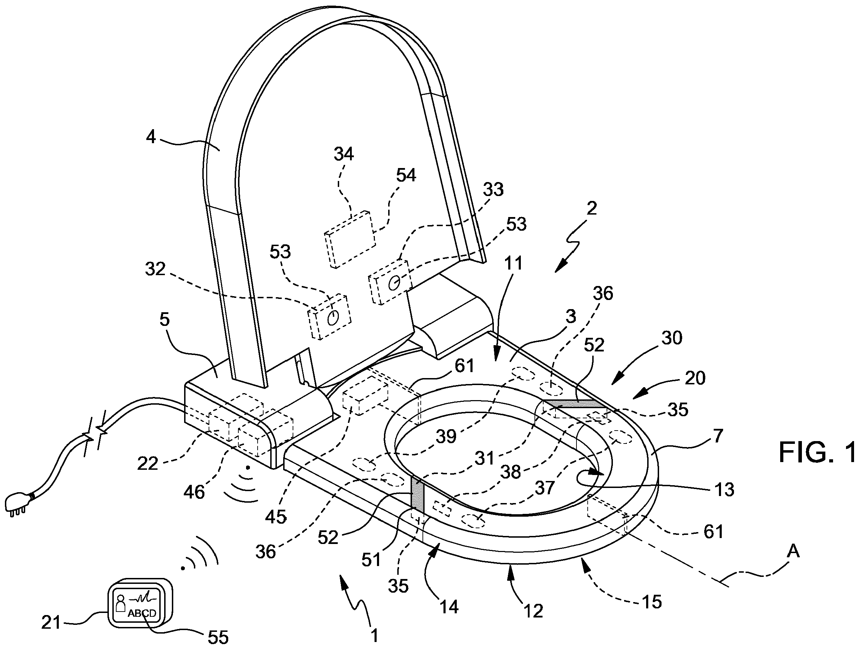

Figure 1 schematically shows a system 1 for detecting biomedical parameters from a subject (a person) seated on a toilet seat (i.e. a seat for a toilet or WC).

The system 1 is integrated in a toilet seat assembly 2 comprising a toilet seat 3, a lid 4, and a support 5 that can be fixed to a sanitary bowl (known and not shown) in any known manner (for example, by means of a pair of bolts or other fastening members) and is hinged to the seat 3 and to the lid 4, for example through respective rotation pins (not shown).

The seat 3 has a substantially annular body 7, which can take various forms, also based on the shape of the bowl on which it is to be mounted. In general, the body 7 extends along a central axis A and has a longitudinally elongated donut shape. Optionally, the body 7 does not form a closed (complete) ring, but is frontally open, i.e. is provided with a radial through cut in a front area of the seat 3.

The body 7 extends between an upper surface 11 and a lower surface 12, opposite to each other; and has a radially inner peripheral edge 13 and a radially outer peripheral edge 14, facing to each other and extending between the upper surface 10 and the lower surface 12.

The body 7 can be a hollow body, in which the upper surface 11 and the edges 13, 14 delimit a substantially annular cavity 15 beneath the surface 11; or the body 7 can be a substantially solid body, provided with one or more cavities 15 formed in the body 7.

The system 1 is configured to perform biometric recognition and biomedical monitoring of users of sanitation facilities, in particular, users of the toilet seat assembly 2, preferably in a continuous way (even though the system 1 can perform the same functions also at predetermined instants, i.e. momentarily).

The system 1 comprises: a sensory unit 20, a control unit 21, and a power supply unit 22.

According to different embodiments of the invention, the sensory unit 20, the control unit 21, and the power supply unit 22 can be differently shaped and configured and variously arranged and positioned with respect to one another and to the components of the toilet seat assembly 2.

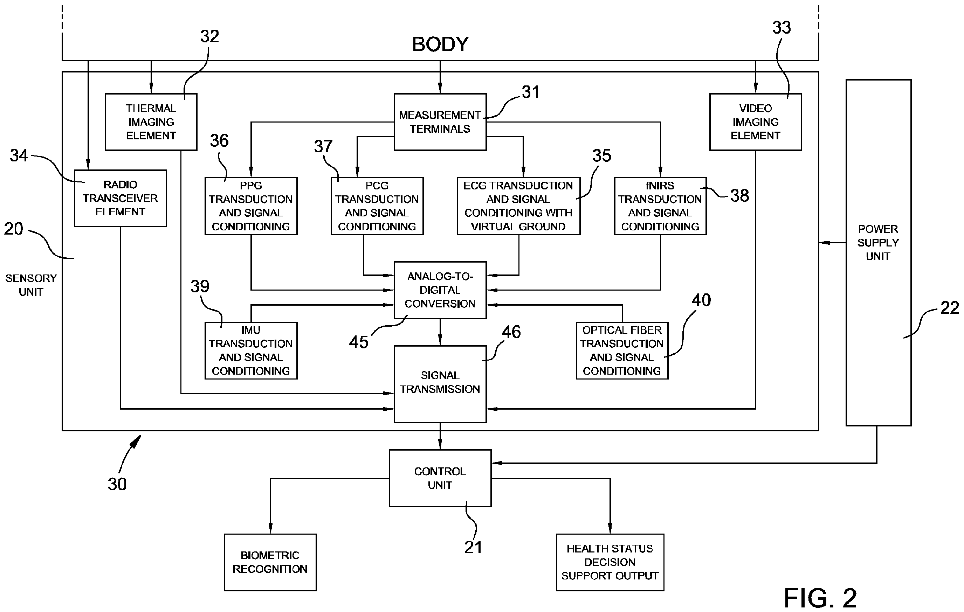

Some exemplary embodiments of the invention are described in details hereinbelow, while the general configuration of the system 1 is shown in Figure 2, which is a block diagram depicting the main components of the system 1, i.e. the sensory unit 20, the control unit 21, and the power supply unit 22.

With reference to both Figures 1 and 2, the sensory unit 20 is configured to detect signals from the user (seated on the toilet seat 3 in a normal position of use) and to process said signals in a form to be transmitted to the control unit 21.

In particular, the sensory unit 20 comprises one or more electronic sensor modules 30 capable of detecting signals from the user and selected (either individually or in any combination) from:
contact measurement module 31;
thermal imaging module 32;
video imaging module 33;
radio transceiver module 34;
ECG (electrocardiography) module 35;
PPG (photoplethysmography) module 36;
PCG (phonocardiography) module 37;
fNIRS (functional near infrared spectroscopy) module 38;
IMU (inertial measurement unit) module 39;
optical fiber module 40.

The sensor modules 30 are configured to detect signals (representative of body parameters) from the user, and/or to process said signals, in particular by transduction and signal conditioning, operating individually or together with other sensor modules 30.

If one or more sensor modules 30 are configured to provide analog signals, the system 1 additionally comprises an analog-to-digital conversion module 45, connected to the sensor modules 30 to convert analog signals provided by the sensor modules 30 to digital signals, if needed.

The system 1 also comprises a signal transmission module 46, configured to receive signals from the sensor modules 30 (possibly converted by the analog-to-digital conversion module 45) and transmit said signals to the control unit 21.

As already mentioned, the sensor modules 30 may include a number of different modules, configured to detect and process respective signals representative of different parameters of the user, and/or to process said signals.

Basically, the sensor modules 30 comprise respective electronic circuits possibly associated with sensors of different types. Depending on the operating principle of the sensors, the sensor modules 30 can be in direct contact with any part of the user's body or not.

The right and/or left legs (in particular, the respective thighs) are the preferred application points in the case where contact is needed, while the back of the trunk and buttocks are the preferred zones in the vicinity of which contactless sensor modules (not requiring direct contact with the user body) are placed, although these are merely illustrative examples.

In particular, the contact measurement module 31 comprises at least one sensor 51 defining a direct interface with the user skin for acquiring ECG, PPG, PCG, and fNIRS signals, which are then processed by the ECG module 35, the PPG module 36, the PCG module 37, the fNIRS module 38.

According to the invention, the sensor 51 is a contact sensor which is positioned, in use, in direct contact with a part of the user's body, as detailed hereinbelow.

The sensors 51 can be based on different operating principles: in particular, the sensors 51 of the contact measurement module 31 operates by detecting the electrical potential difference measured between two sensor terminals 52 in contact with the body (skin) of the user; in addition, the contact measurement module 31 can also include sensors capable of detecting changes in the electromagnetic field measured using a capacitive element, or capable of measuring mechanical actions of any type (such as piezoelectric, or pressure sensors, etc.), without necessarily requiring a direct contact with the user body.

The contact measurement module 31 can be either integrated in the sensory unit 20 (possibly together with other modules 30) or consist of an independent module, connected to the remaining modules 30 of the sensory unit 20 by wires or cables.

In particular, the contact measurement module 31 is connected to the ECG module 35, the PPG module 36, the PCG module 37, and the fNIRS module 38.

The thermal imaging module 32, the video imaging module 33, and the radio transceiver module 34 typically work in proximity to but not in direct contact with the skin of the user.

In particular, the thermal imaging module 32 and the video imaging module 33 include respective cameras or other similar imaging elements 53 configured to acquire images (thermal images or optical images respectively) from the user. The thermal imaging module 32 and the video imaging module 33 (with the respective imaging elements 53) are advantageously housed in the lid 4, in proximity of a lower edge thereof hinged to the support 5 and close to the toilet seat 3, or in another structure of the toilet seat assembly 2 or other associated installations.

The output of the thermal imaging module 32 and the video imaging module 33 allows a further enhancement of the method of the invention for biometric recognition and/or biomedical monitoring. In fact, image acquisition and processing can be used by the system 1 to capture footage from the body of the user and/or for analysis of substances excreted by the user.

The radio transceiver module 34 comprise one or more radio transceiver elements 54 configured to emit and/or receive radio waves in the GHz range, so as to detect mechanical displacements associated with the cardiovascular activity of the body. The radio transceiver elements 54 of the radio transceiver module 34 can be fitted on the toilet seat 3 or the lid 4.

The ECG module 35, the PPG module 36, the PCG module 37 and the fNIRS module 38 are all connected to the contact measurement module 31 to process data received therefrom.

Each of the ECG module 35, the PPG module 36, the PCG module 37 and the fNIRS module 38 comprise an electronic circuit configured to perform transduction and signal conditioning.

The electrical voltages associated with the cardiovascular system activity are measured by using the ECG module 35, which receives ECG signals from the contact measurement module 31, acquired by the pair of terminals 52 of the sensors 51, and provides to the transduction and conditioning with virtual ground of the ECG signals.

In particular, the ECG module 35 is configured to perform signal transduction and conditioning with virtual ground of ECG data.

In more details, the ECG module 35 is configured to perform the detection and conversion of physical parameters, detected by the contact measurement module 31, into signals and to filter and amplify said signals so that the signals can be treated as an electrical quantity. Preferably, the electronic circuit of the ECG module 35 includes an electromagnetic noise filter, compatible with the transmission of the signals through a wireless connection; and an amplifier. As a result, the electrical quantity is a clean and high definition representation of the physical quantity, free of external noise.

Preferably, the ECG module 35 is equipped with a band pass filter, particularly advantageous in the present invention by having a passing band between 0.5 and 40Hz, so as to eliminate the need for traditional notch filters, simplifying the physical construction of the module while allowing an adequate separation between the ECG signal and parasite signals such as motion artifacts, baseline wander, muscle signals, power line interference, among others.

Amplification occurs with a gain between 10 and 40000, allowing a significant increase in the definition of the collected signal (in the order of uV, mV or V), making the tenuous ECG signals more immune to external noise, and enabling a sufficient definition for the biometric recognition and/or biomedical monitoring method to operate.

According to the invention, the ECG module 35 is particularly advantageous insofar the sensors 51 of the contact measurement module 31 operate in direct contact with the user body: in fact, according to a preferred aspect of the invention, the ECG module 35 has an electronic circuit configured so as to generate a virtual ground, i.e. to provide a virtual reference voltage, enabling the use of only two measurement terminals and eliminating the need for the traditional third electrode that collects the real ground, indispensable for the acquisition of ECG signals by traditional methods.

The electronic circuit of the ECG module 35 provides a stable reference voltage potential to the signal conditioning circuit, without requiring a direct connection to reference potential; for example, the virtual ground can be implemented using a voltage divider, a rail splitter or any other analogous circuit.

The PPG module 36 is configured to perform transduction and signal conditioning of PPG (photoplethysmography) data. In particular, the PPG module 36 is configured to perform the conversion and detection of physical parameters received from the contact measurement module 31 into signals, and to filter and amplify the resulting signals so that the signals can be treated as an electrical quantity. Preferably, also the module 36 of PCG transduction and signal conditioning includes an electromagnetic and mechanical noise filter, compatible with the vibrations transmitted to the contact measurement module 31 and/or with the transmission of the signals through a wireless connection, resulting in that the electrical quantity is a clean and high definition representation of the physical quantity, free of external noise.

The fNIRS module 38 is configured to measure optical processes associated with the cardiovascular activity of the body. According to the invention, the fNIRS module 38 is directly integrated in the toilet seat 3, in order to promote a contact or line-of-sight proximity with the skin of the user.

The fNIRS module 38 is configured to perform transduction and signal conditioning of fNIRS data. In particular, the fNIRS module 38 is configured to perform the conversion and detection of the physical quantity through the contact measurement module 31, filters the resulting signal, and amplifies it so that it can be treated as an electrical quantity. Also the module 38 preferably includes an electromagnetic and optical noise filter, compatible with the luminous interference transmitted to the contact measurement module 31 and/or with the transmission of the signals through a wireless connection. As a result of the operation of the module, the electrical quantity is a clean and high definition representation of the physical quantity, free of external noise.

The fNIRS module 38 includes at least one optical emitter and one optical receiver, although it can include more elements working at different luminous wavelengths to enable a more diverse observation of the vascular dynamics, as a way to obtain more reliable biometric recognition and/or results of biomedical monitoring.

Similarly to other modules, also the fNIRS module 38 has an electronic circuit configured to filter and amplify the signals, in order to produce a more suitable representation of the physical quantity. Also in this case the filter is preferably a band pass filter, particularly advantageous in the present invention, which, by having a high pass cutoff frequency of 0.5Hz and a selectable low pass cutoff frequency of between 5Hz and 1kHz, enables a wide dynamic range, simplifying the physical construction of the device while allowing an adequate separation between the underlying physiological processes. The amplification has a gain between 1 and 40000, allowing the increase in the definition of the collected signal, making the tenuous fNIRS signals more immune to external noise. The intensity of the emitted light can also be reduced or increased, in order to improve the definition of the tenuous fNIRS signals and make the output more immune to external noise. The overall design of this sensor enables a sufficient definition for the biometric recognition and/or biomedical monitoring method to operate.

The mechanical displacements associated with the cardiovascular activity of the body, propagated onto the toilet seat cover, are measured using the PCG module 37, the IMU module 39, and/or the optical fiber module 40. All said modules are advantageously integrated in the toilet seat 3 or another structure of the sanitary facilities and operates without needing a direct contact with the user (as indeed required for the signal acquisition by using traditional methods).

Each of the PCG module 37, IMU module 39, and optical fiber module 40 has an electronic circuit configured to perform the filtering and amplification of the respective signals, producing a more suitable representation of the physical quantity by filtering and amplifying.

Preferably, the electronic circuit comprises a band pass filter, particularly advantageous in the present invention which, by having a high pass cutoff frequency of 0.5 Hz and a selectable low pass cutoff frequency of between 50 Hz and 50 kHz, enables a wide dynamic range, simplifying the physical construction of the device while allowing an adequate separation between the PCG, IMU and/or optical fiber signal and parasite signals such as motion artifacts or baseline wander, among others.

The circuit also include an amplification circuit configured to provide an increase between 1 and 40000, allowing a significant increase in the definition of the collected signal, making the tenuous PCG, IMU and/or optical fiber signals more immune to external noise, and enabling a sufficient definition for the biometric recognition and/or biomedical monitoring method to operate.

Preferably, the PCG module 37, the IMU module 39, and/or the optical fiber module 40 are positioned close to the point of interface with the user (i.e. near the terminals 52), so significantly reducing the appearance of parasite signals.

The PCG module 37 and/or the IMU module 39 can be integrated in the contact measurement module 31.

The optical fiber module 40 can consist of one or more polymer optical fiber Bragg grating elements placed around the toilet seat cover structure.

In more details, the IMU module 39 is configured to perform transduction and signal conditioning of IMU data. In particular, the IMU module 39 is configured to autonomously perform the conversion and detection of the physical quantity, to filter the resulting signal, and to amplify the signal so that it can be treated as an electrical quantity. Preferably, the module 39 of IMU transduction and signal conditioning includes an electromagnetic and mechanical noise filter, compatible with the vibrations transmitted to the contact measurement module 31 and/or with the transmission of the signals through a wireless connection. As a result of the operation of this module, the electrical quantity is a clean and high definition representation of the physical quantity, free of external noise.

The optical fiber module 40 is configured to perform transduction and signal conditioning of optical data. In particular, the module 40 is configured to perform the conversion and detection of the physical quantity autonomously, filter the resulting signal, and amplify it so that it can be treated as an electrical quantity. The module 40 preferably includes an electromagnetic and optical noise filter, compatible with the luminous interference transmitted to the contact measurement module 31 and/or with the transmission of the signals through a wireless connection. As a result of the operation of this module, the electrical quantity is a clean and high definition representation of the physical quantity, free of external noise.

The analog-to-digital conversion 45 module is configured to transform the electrical quantity, obtained through the ECG module 35, the PPG module 36, the PCG module 37, the fNIRS module 38, the IMU module 39, the optical fiber module 40, in an adequate digital representation manageable in the control unit 21.

The module 45 includes an electronic circuit that integrates a quantization element and an analog-to-digital converter. The quantization element is a component that maps the voltage or current to a set of bits (also known as resolution), which in the case of the present invention preferably ranges between 8 and 64 bits. The analog-to-digital converter is a component that, at regular and pre-defined time intervals, collects a sample, which is then quantized. In the case of the present invention, the frequency at which the samples are collected (sampling rate) can range between 250Hz and 16kHz, which in samples collected per unit of time corresponds respectively to 250 and 16000 samples per second.

The signal transmission module 46 is configured to transmit the digital representation generated by the analog-to-digital conversion module 45 to the control unit 21. The signal transmission module 46 and the control unit 21 may communicate with each other, for example, by a cabled or wireless connection, or by means of electrical tracks on a printed circuit board (for example, if the modules are integrated).

The signal transmission module 46 can operate by using several methods. Preferably, the module 46 uses a wireless channel based on existing protocols such as Bluetooth, WiFi, ZigBee, Narrow Band IoT, SigFox, LoRa, ANT, GPRS, and/or 3G/4G/5G, although other protocols can also be admissible.

Alternatively, the transmission can also be performed in a cabled manner, which in the present invention is also advantageous since it can be performed through more conventional interfaces, such as USB, COM/RS232, GPIO pins, tracks in a printed circuit board, direct connection to the Rx/Tx pins on a micro controller, and others.

The data can be transmitted from any one of the analog-to-digital conversion module 45, the thermal imaging element 32, the video imaging element 33, and/or from the radio transceiver module 34, as plain raw data, which is the general approach. Nevertheless, the data can also be transmitted as a transformed version of the raw data, computed using non-reversible functions (e.g. dissimilarity metrics) while preserving the informative content of the original data to the best extent possible.

The control unit 21 can be any type of electronic device suitable for digital processing and presentation of signals and data.

The control unit 21 can be a dedicated control unit, for example a microcontroller unit, either integrated in any part of the toilet seat assembly 2, for example in the toilet seat 3, or located in a remote position; the control unit 21 can also be the same central control unit of the toilet or other sanitation facility in which the system 1 is integrated; the control unit 21 can also be integrated in a portable electronic device such as a tablet computer, a mobile phone, etc.

In any case, the control unit 21 is configured to process data received from the sensory unit 20 and collected in real time by the sensory unit 20 from the user, and to extract from said data information representative of the conditions of the user so as to perform the biometric recognition of the user and evaluate the health status of the user.

Preferably, the control unit 21 is associated with a display unit 55 configured to provide a graphical presentation and/or representation of the signals processed by the control unit 21, of the result of the recognition process, and/or of the result of the analysis of the signals. For example, the display unit 55 can be the screen of the dedicated electronic device in which the control unit 21 is integrated, or the screen of a smartphone or tablet computer or other electronic device, the screen of the central console of the toilet, or any other similar device.

Operation of the control unit 21, implementing the method of the invention, is described in details hereinbelow.

The power supply unit 22 can be any electric and/or electronic equipment, with galvanic isolation, suitable for providing an input voltage with which the system 1 can operate. For example, the power supply unit 22 can include one or more of: a connection to the mains; a battery (such as a Lithium-Polymer battery) or another energy storage device; a wireless receiver capable of receiving energy from a dedicated wireless emission source; a hydraulic generator installed in the toilet (or in another structure where water flows); a receiver capable of receiving energy from WiFi or other radio frequency signals emitted from an external emitter; a solar panel installed on the toilet or in another location in the sanitary facility; etc.

In use, the system 1 operates, implementing the method of the invention, as follows.

The sensor modules 30 of the sensory unit 20 detect respective signals from the user.

The signals detected and measured by the contact measurement module 31 are transmitted to one or more modules selected from: the ECG module 35, which performs transduction and signal conditioning with virtual ground and filtering and amplification of the ECG signals; the PPG module 36, which performs transduction and signal conditioning and filtering and amplification of the PPG signals; the PCG module 37, which performs transduction and signal conditioning and filtering and amplification of the PCG signals; the fNIRS module 38 which performs transduction and signal conditioning and filtering and amplification of the fNIRS signals.

If needed, all the above mentioned modules transmit data to the analog-to-digital conversion module 45 for the conversion into digital signals.

Also the signals from the IMU module 39, the optical fiber module 40, the thermal imaging element 32, the video imaging element 33, and the radio transceiver element 34, possibly converted in the analog-to-digital conversion module 45 if needed, are sent to the transmission module 46 and transmitted to the control unit 21.

The control unit 21 processes the signals received from the sensory unit 20 and produces a result (decision) about the biometric recognition of the user and/or about the health status and biomedical monitoring of the user.

The result produced by the control unit 21 is displayed by the display unit 55.

The control unit 21 is configured so as to implement a method that adjusts the way in which it operates, enabling the extraction of representative information from the signals collected from the user, the corresponding biometric recognition, the result of the biomedical assessment, and/or the production of a technical effect according to the produced biometric recognition and/or health status decision support output.

The process is preferably performed in a continuous way, that is ensuring that the biometric recognition and/or biomedical monitoring of users of sanitation facilities occurs uninterruptedly while the system 1 is being used, although it can also be performed in a momentary way.

The control unit 21 is also configured so as to implement a method for customization of its settings as a result of the biometric recognition of the user. In particular, the control unit 21, after identifying the user on the basis of the data received from one or more sensor modules 30, for example on the basis of ECG data processed by the ECG module 35, then commands activation of further modules sensors 30, to integrate customized biomedical analysis for the specific identified user.

The method implemented by the control unit 21 is particularly advantageous in that the method provides not only the digital representation of parameters produced by the modules 30; but it is also capable of extracting from the sensory unit 20 relevant information about the biometric recognition and/or biomedical monitoring of the user, even using signals that are collected at the thighs, which, for all the constituents of the sensory unit 20, are not the conventional operating locations.

In order to carry out the biometric recognition of the user, the control unit 21 includes (or has access to) a memory where a database of registered users is recorded. The database is initially compiled, for example, by using one or a combination of the modules 30 that constitute the sensory unit 20. The control unit 21 either integrates a database of known users, or communicates with a central server that stores that information remotely.

The control unit 21, in order to provide the biometric recognition of the user, receives data from the sensory unit 20, and process and compare the data with the stored data so as to identify the user.

The biometric recognition method used in the present invention is particularly advantageous given that it can produce a decision on the identification or authentication of the user in a continuous mode, that is, guaranteeing that the biometric recognition occurs uninterruptedly during the time in which the user interacts with the device, or just upon request, that is, in a momentary way, where the recognition is optionally effected and repeated at predetermined time intervals, spaced by several minutes, hours, or days. The method applies a set of machine learning and/or artificial intelligence methods, which use one or more of the signals collected by the sensory unit 20, in raw form or as an alternative representation generated from the representative information extracted from them, and that matches the resulting information with the patterns previously stored in the enrolled users database.

The recognition process comprises, in sequence, a pre-processing step, in which the signal received from the sensory unit 20 is pre-processed to provide a pre-processed signal; an extraction step, in which data representative of the user are extracted from the pre-processed signal; and a final classification step, in which a decision is produced identifying the user.

In the pre-processing step, an additional digital filtering step is implemented which complements those performed in the sensory unit 20.

The extraction step automatically learns a representation (e.g. by means of an artificial neural network) or performs the segmentation of the collected signals (or, in alternative, just a few components of the same). Representative information about the complexes can also be extracted for each user, such as latencies, amplitudes, spectral density and others; furthermore, the average of several signal waveforms or of the extracted information can also be used. These are also particularly advantageous properties of the present invention.

In the classification step, the representative information is compared with the data in the database, to determine or validate the identity of the user by using an Artificial Neural Network (ANN), or, alternatively, a nearest neighbor (k-NN) approach with Euclidean distance as similarity metric, or Support Vector Machines (SVM). Still, other classification methods are admissible in the context of the present invention.

Finally, a decision about the biometric recognition of the user and/or about the health status support is produced in the control unit 21.

As already mentioned, the control unit 21 preferably provides the results/decisions by the display unit 55, in particular by means of the on-board display or a projection device, a mobile app, a cloud-based portal, an online database, just to name a few examples, although other analogous methods are admissible.

The result produced by the control unit 21 can have different technical effects, which include but are not limited to: identification of the user of the toilet seat, verification of the identity of the user of the toilet seat, presentation of decisions and results of the biomedical monitoring analysis to the user, customization of settings according to the perceived user, issue of notifications pertaining the health status of the user.

For example, but not necessarily, the method implemented in the control unit 21 uses, as input data received from the sensory unit 20, data, in raw or transformed form, selected from: ECG waveform, PCG waveform, fNIRS waveform (in the different luminous wavelengths), IMU waveform, optical fiber waveform, radio transceiver absorption waveform, thermal imaging footage, and/or standard video footage. Based on this data, the method can output, amongst other parameters: user identity, usage frequency, arrhythmia detection, optical-optical pulse transit time, electrical-optical pulse transit time, force exerted in the toilet seat, pressure mapping, approximated weight, respiration, stool analysis, approximated fecal weight, approximated fecal consistency, urine analysis, atrial fibrillation, blood oxygenation, blood flow, approximated blood pressure, body fat, heart rate, heart rate variability, temperature, and/or posture.

The device is designed for continuous operation and, as such, it can be used to assess the user's health status as an extension of the normal everyday practices that the user already has within sanitary facilities.

The device is designed for continuous operation and, as such, it can be used to assess the user's health status as an extension of the normal everyday practices that the user already performs within sanitary facilities.

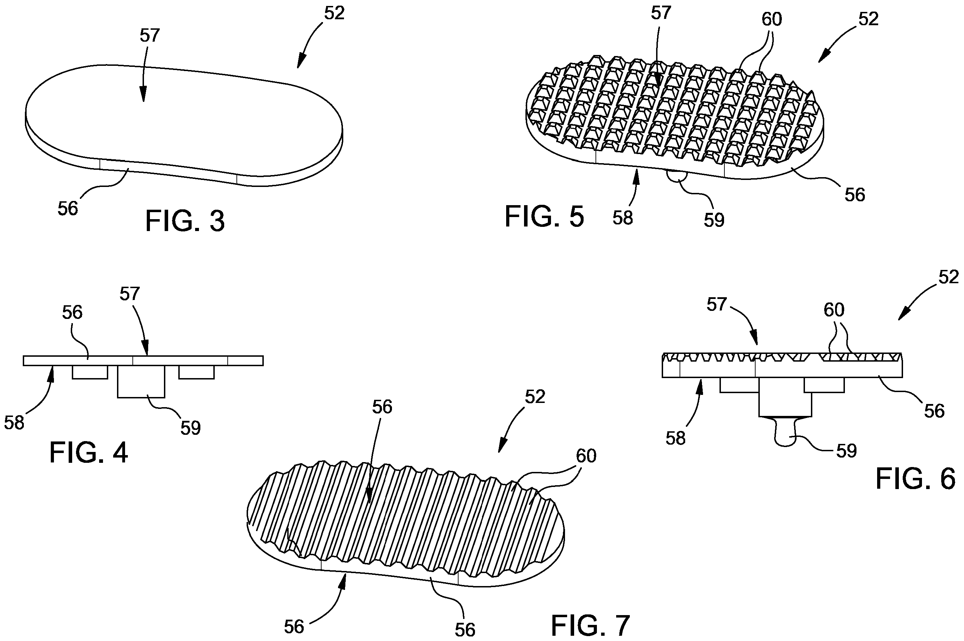

Figures 3 to 7 show preferred embodiments of the sensor terminals 52 of the contact measurement module 31.

With reference to Figures 3-4, each terminal 52 comprises a substantially flat pad 56 (which can have different shape and size), having a conductive top surface 57 for contacting the user body and a bottom surface 58, opposite to the top surface 57, provided with connectors 59 for electrical connection. The terminals 52 are housed in the toilet seat 3 and are positioned so as the top surface 57 is substantially flush with the upper surface 11 of the toilet seat 3. The terminals 52 are positioned so as to contact, when the user is seated on the toilet seat 3 in a normal position of use, the left and right thighs respectively of the user.

Advantageously, the conductive top surface 57 of each terminal 52 covers the whole respective section of the toilet seat 3 (right or left sections respectively), extending substantially between the radially inner peripheral edge 13 and the radially outer peripheral edge 14 of the toilet seat 3.

According to a preferred aspect of the invention, the terminals 52 or at least a portion thereof comprising the top surface 57 (for example, the pad 56) are advantageously made of non-metallic materials, such as conductive coatings, conductive ceramics, conductive polymer materials (plastic), indium tin oxide (ITO), or other materials that are functionalized to eliminate the need to use any kind of explicitly metallic element that modifies the typical appearance of a toilet seat and/or the need for conductive gel or paste.

For example, in a preferred embodiment, the sensor 51 has terminals 52 consisting of a ceramic core functionalized with an outer ITO coating, so as there are no metal parts (pads) and there is no need for using conductive gel or paste for a good interface with the user skin.

According to the invention, only two terminals 52 are needed in order to detect ECG signals from the user body.

In fact, the terminals 52 allow the measurement of ECG signals between the left and right thighs.

The contact measurement module 31 is particularly advantageous in that, in addition to the measurement function, the terminals 52 which are in contact with the body of the user can also perform additional operations: for example, the terminals 52 can be configured so as to generate heat and/or electrical stimulation, so as to act as heating pads and/or as electrical stimulation pads thus providing an actuation function on the user body.

Moreover, as shown in Figures 5 to 7, in some preferred embodiments the terminals 52 are provided with projections 60, protruding from the top surface 57 of the pad 56 to increase the contact between the terminals 52 (and thus the contact measurement module 31) and the skin of the user, as a way of overcoming natural barriers of the body, such as hair (in fact, body hair constitutes a barrier to the conduction of electrical signals).

The projections 60 can be variously shaped and arranged according to different patterns. For example, in the embodiment of Figures 5-6 the pad 56 is provided with a grid of projections 60 having a truncated sphere, truncated cone or truncated pyramid shape, arranged in rows. In the embodiment of Figure 7 the pad 56 is provided with a series of transversal projections 60 in the form of ribs parallel to one another and defining a series of alternate protruding concavities and/or convexities.

The physical position of the ECG module 35 with respect to the contact measurement module 31 and the terminals 52 is also particularly advantageous according to the invention. Although several other configurations are admissible in the context of the invention, the ECG module 35 is more advantageously arranged in close proximity of the point of interface with the user (i.e. in close proximity of the terminals 52) so as to greatly minimize the appearance of parasite signals. In traditional signal acquisition methods, ECG modules are usually placed far away from the point of interface with the user, making the cabled connection work as an antenna that captures several surrounding noise sources.

If the contact measurement module 31 comprises only two terminals 52, the two terminals 52 are positioned on opposite sides of the toilet seat 3, i.e. on opposite sides of the central axis A, in positions corresponding to the left and right thighs of the user seated on the toilet seat 3. The two terminals 52 are separated by insulating elements 61 positioned between the terminals 52 in the toilet seat 3, for example along the central axis A, to ensure that there is no electrical path between the two terminals 52.

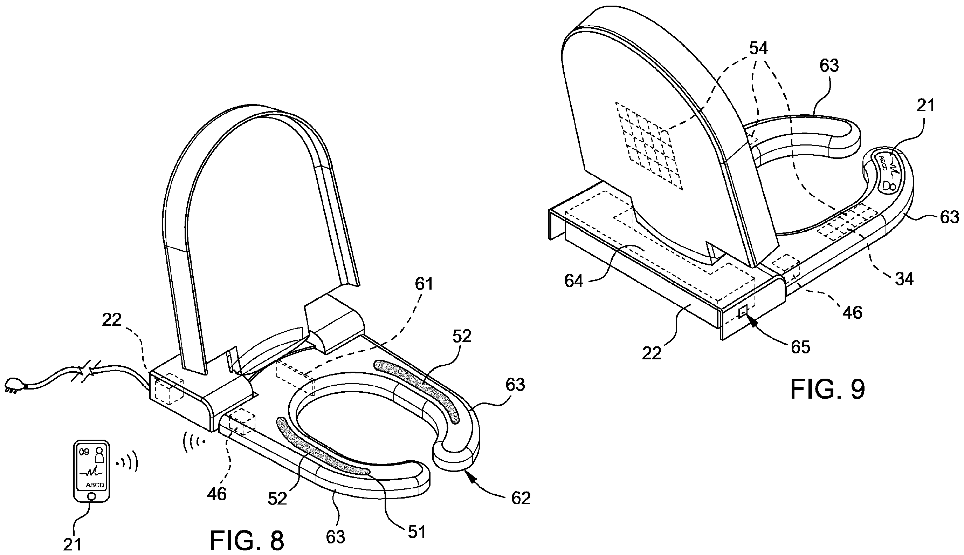

If the toilet seat 3, as shown for example in Figure 8, has a front opening 62 that divides two substantially parallel arms 63 of the annular body 7, only one insulating element 61 is needed at the rear portion of the annular body 7, opposite to the front opening 62, where the arms 63 are joined.

If more than two terminals 52 are used, each terminal 52 needs to be insulated from other contiguous terminals 52. The use of more terminals 52 results in a larger area available for contact with the user and favors the acquisition of very high definition and low noise signals, without affecting the ability to adequately clean neither the surface nor the natural aesthetics of the toilet seat.

It remains understood that the sensory unit 20, the control unit 21 and the power supply unit 22, as well as the sensor modules 30, can be variously arranged with respect to one another.

In a preferred embodiment, the sensory unit 20 and the control unit 21 are integrated in one another.

In other embodiments, however, the sensory unit 20 and the control unit 21 are separated from one another and arranged in different positions; the signal transmission module 46 ensures communication between the sensory unit 20 and the control unit 21.

For example, in an embodiment the sensory unit 20 and the transmission module 46 are housed in the toilet seat 3 or in the lid 4, while the control unit 21 is in a remote position, being for instance the toilet seat central console or a dedicated device.

It remains understood, however, that all the previously described examples do not limit the scope of the invention as defined by the appended claims. In particular, the control unit 21 can be defined by a plurality of different devices, not only a dedicated hardware device, specifically designed as a component of the system 1, but also by an existing hardware device, such as a portable electronic device (smartphone, smart watch, tablet, notebook, portable computer, etc.) or any kind of computer or processing unit.

In the embodiment of Figure 8, the control unit 21 is integrated in a smartphone. The contact measurement module 31 comprises two terminals 52 arranged on opposite sides of the toilet seat 3, i.e. of the central axis A. In this case, the terminals 52 have a longitudinally elongated shape along respective lateral portions of the annular body 7 of the toilet seat 3.

The terminals 52 can have the features previously described.

In the embodiment of Figure 9, the control unit 21 is housed in the toilet seat 3, in particular in a front portion of one of the arms 63; and the power supply unit 22 comprises a battery 64 housed in the support 5 and having a connector 65 (e.g. a USB connector) for recharging the battery 64.

In this embodiment, the radio transceiver module 34 comprises a plurality of radiotransceiver elements 54 arranged in different positions on the toilet seat 3 and/or the lid 4.

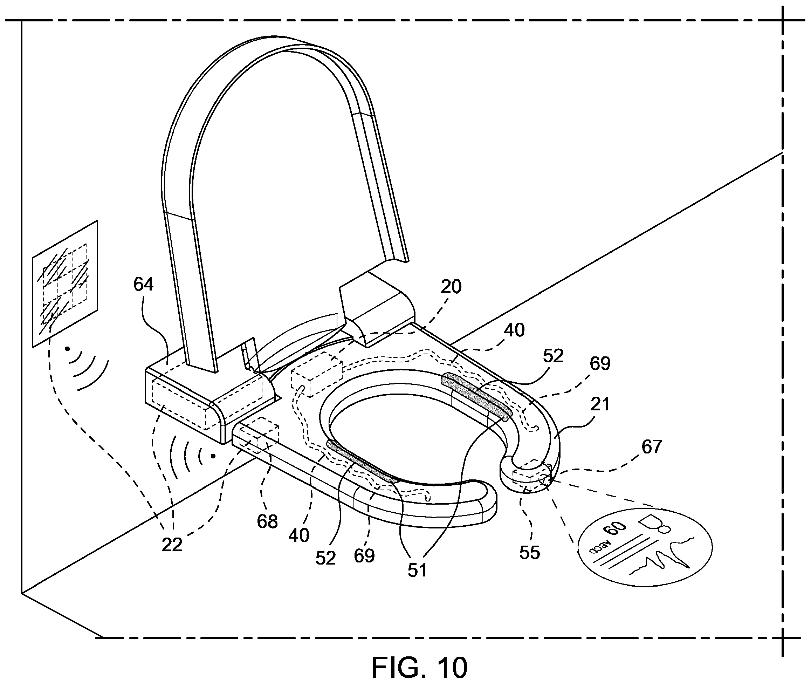

In the embodiment of Figure 10, the control unit 21 is associated with a display unit 55 comprising a projection device 67 which is configured to project information elaborated by the control unit 21 on any kind of screen, for example directly on the floor in front of the toilet seat assembly 2.

In this embodiment, the power supply unit 22 additionally includes both a battery 64 and a wireless and/or WiFi receiver 68 capable of receiving energy from an external dedicated wireless emission source or from an external WiFi or other radio frequency signals emitter.

The terminals 52 of the contact measurement module 31 have a longitudinally elongated shape along respective lateral portions of the annular body 7 of the toilet seat 3; the top surface 57 of each terminal 52 extends to cover both a portion of the upper surface 11 of the toilet seat 3, and a portion of the inner peripheral edge 13 of the toilet seat 3.

The optical fiber module 40 comprises optical fiber elements 69, for example polymer optical fiber Bragg gratings, placed on the toilet seat 3 and in particular along opposite lateral portions (on opposite sides of the central axis A) of the toilet seat 3.

Of course, all the embodiments described above and any individual feature or component thereof can be combined with one another.

Finally, it is understood that further modifications and variants may be made to the method and system described and illustrated herein without departing from the scope of the appended claims.

## Claims

1. A method for biometric recognition, in particular of a user of sanitation facilities, comprising the steps of:
- providing a contact measurement module (31) having at least one sensor (51) defining a direct interface with the user body for acquiring ECG signals from the user and comprising a pair of terminals (52) to be positioned in direct contact with the user body;
- detecting an electrical potential difference measured between said two sensor terminals (52) of the contact measurement module (31) ;
- processing in continuous mode the signals detected by the contact measurement module (31) to provide ECG data of the user;
- performing the biometric recognition of the user by comparing said ECG data with data stored in a database of known users, so as to identify a current user.

2. A method according to claim 1, wherein the biometric identification of the user is performed in a continuous way, by continuously acquiring signals from the user and processing said signals along a period of time in which the user is in contact with the terminals (52).

3. A method according to claim 1 or 2, wherein the biometric identification of the user comprises, in sequence, a pre-processing step, in which the signal received from the sensory unit (20) is pre-processed to provide a pre-processed signal; an extraction step, in which data representative of the user are extracted from the pre-processed signal; and a final classification step, in which a decision is produced identifying the user.

4. A method according to one of claims 1 to 3, comprising a step of displaying the result of the recognition.

5. A method according to one of claims 1 to 4, comprising a step of acquiring and processing one or more of:
thermal images and/or video images of at least a part of the user body;
mechanical displacements associated with the cardiovascular activity of the body by detecting radio waves in the GHz range;
PPG (photoplethysmography) data;
PCG (phonocardiography) data;
fNIRS (functional near infrared spectroscopy) data;
IMU (inertial measurement unit) data;
optical fiber data.

6. A method according to one of claims 1 to 5, comprising a step of customizing the information provided as a result of the method, by automatically adjusting the operation of the method according to the identity of the user as recognized by the method.

7. A system for biometric recognition of a user of sanitation facilities, in particular a subject seated on a toilet seat, comprising: a toilet seat assembly (2) comprising at least a toilet seat (3); a sensory unit (20) comprising one or more electronic sensor modules (30) configured to detect data from a user seated on the toilet seat (3) in a normal position of use; a control unit (21) connected to the sensory unit (20) to receive data therefrom; and a power supply unit (22) to supply power to the sensory unit (20) and the control unit (21); wherein the sensor modules (30) comprise at least a contact measurement module (31) having at least one sensor (51) defining a direct interface with the user body for acquiring ECG signals from the user; and an ECG module (35), configured to process ECG signals received from the contact measurement module (31); **characterized in that** the control unit (21) is configured to process, operating in continuous mode, data continuously received from the ECG module (35) of the sensory unit (20) and collected in real time by the sensory unit (20) from the user, so as to provide ECG data of the user and perform the biometric recognition of the user by comparing said ECG data received from the ECG module (35) with a database of registered users.

8. A system according to claim 7, wherein the sensor (51) of the contact measurement module (31) comprises a pair of terminals (52) positioned on the toilet seat (3) so as to be in direct contact with respective thighs of the user seated on the toilet seat (3) in a normal position of use; the sensor (51) being configured to detect an electrical potential difference measured between said two sensor terminals (52) in contact with the body of the user; the ECG module (35) being configured to perform signal transduction and conditioning with virtual ground of the ECG signals received from the contact measurement module (31) .

9. A system according to claim 8, wherein the ECG module (35) has an electronic circuit configured to perform detection and conversion of physical parameters, acquired by the contact measurement module (31), into signals and to filter and amplify said signals so that said signals can be treated as an electrical quantity to be transmitted to the control unit (21); the electronic circuit of the ECG module (35) being configured so as to generate a virtual ground signal, i.e. to provide a virtual reference voltage, enabling the use of only two terminals (52) for the acquisition of ECG signals.

10. A system according to any one of claims 7 to 9, wherein the ECG module (35) is arranged in proximity of the terminals (52) of the contact measurement module (31) defining the interface with the user, in particular on the toilet seat (3).

11. A system according to any one of claims 7 to 10, wherein the contact measurement module (31) comprises only two terminals (52), positioned on opposite sides of the toilet seat (3) and of a central axis (A) thereof, in positions corresponding to the left and right thighs of the user seated on the toilet seat (3); and wherein the two terminals (52) are separated by one or more insulating elements (61) positioned between the terminals (52) in the toilet seat (3), for example along the central axis (A).

12. A system according to any one of claims 7 to 11, wherein the sensory unit (20) further comprises one or more electronic sensor modules (30) selected from:
thermal imaging module (32);
video imaging module (33);
radio transceiver module (34);
PPG (photoplethysmography) module (36);
PCG (phonocardiography) module (37);
fNIRS (functional near infrared spectroscopy) module (38);
IMU (inertial measurement unit) module (39);
optical fiber module (40);
and wherein the control unit (21) is configured to process data received from the EGC module (35) and also from at least one other sensor module (30), and to compare all the data with the database of registered users to identify the user.

13. A system according to any one of claims 7 to 12, wherein the control unit (21) is a dedicated control unit, for example a microcontroller unit, either integrated in any part of the toilet seat assembly (2), for example in the toilet seat (3), or located in a remote position; or the control unit (21) is integrated in another central control unit of a toilet or other sanitation facility in which the system (1) is integrated; or the control unit (1) is integrated in a portable electronic device such as a tablet computer, a mobile phone, etc.

14. A system according to one of claims 7 to 13, wherein the power supply unit (22) includes one or more of: a connection to the mains; a battery or another energy storage device; a wireless receiver capable of receiving energy from a dedicated wireless emission source; a hydraulic generator installed in the toilet or in another structure where water flows; a receiver capable of receiving energy from WiFi or other radio frequency signals emitted from an external emitter; a solar panel.

15. A system according to one of claims 7 to 14, wherein each terminal (52) of the contact measurement module (31) has a conductive top surface (57) for contacting the user body and positioned so as to be substantially flush with the upper surface (11) of the toilet seat (3); the pair of terminals (52) being positioned so as to contact, when the user is seated on the toilet seat (3) in a normal position of use, the left and right thighs respectively of the user; and wherein the terminals (52) or at least a portion thereof comprising the top surface (57) are made of non-metallic materials, such as conductive coatings, conductive ceramics, conductive polymer materials, indium tin oxide (ITO).

16. A system according to claim 15, wherein the terminals (52) are provided with projections (60), protruding from the top surface (57) to increase the contact between the terminals (52), and thus the contact measurement module (31), and the skin of the user.
